# EUROPÄISCHE PATENTSCHRIFT

(11) **EP 1 010 461 B1**
(45) Veröffentlichungstag und Bekanntmachung des Hinweises auf die Patenterteilung: **03.07.2002**
(21) Anmeldenummer: 98123519.5
(22) Anmeldetag: 15.12.1998
(51) Int. Cl.: B01J 2/10, A61K 35/78, F26B 7/00

(54) **Verfahren zur Herstellung von Arzneimittelvorprodukte für Tabletten, Pellets und Dragees**
Process for the preparation of medicament precursors for tablets, pellets or pills
Procédé pour la préparation des precurseurs de medicaments pour comprimés, granules et dragées

(43) Veröffentlichungstag der Anmeldung: 21.06.2000
(73) Patentinhaber: Bionorica AG, 92318 Neumarkt (DE)
(72) Erfinder: Joseph, Heinz-Walter, Dr., 92348 Berg (DE)
(74) Vertreter: Schwabe - Sandmair - Marx

(56) Entgegenhaltungen:
- EP-A- 0 753 306
- WO-A-91/06365
- WO-A-97/23232

## Beschreibung

Die vorliegende Erfindung betrifft ein Verfahren zur Herstellung fließfähiger, pressfertiger Arzneimittelvorprodukte zur Tabletten-, Dragee-, und Pelletsherstellung aus Pflanzendrogen, welche Vorprodukte ohne weitere Zwischenstufen zur Tablettierung verwendet werden können, sowie die Arzneimittelvorprodukte als solche. Insbesondere betrifft sie ein Verfahren, welches die Nachteile der herkömmlichen Feuchtgranulation vermeidet, ohne auf die Direkttablettierung zurückgreifen zu müssen.

Herkömmliche Verfahren zur Herstellung von Tabletten aus getrockneten Pflanzendrogen sind zum einen die Feuchtgranulation, zum anderen die Direkttablettierung. Da die Direkttablettierung den Einsatz vorbehandelter bzw. vorgranulierter Substanzen sowie entsprechend vorbehandelter Hilfs- und Trägerstoffe erfordert, wird aus Kostengründen meist auf die Feuchtgranulation zurückgegriffen. Hierbei werden die Wirkstoffe, also die getrockneten Pflanzendrogen und gegebenenfalls Hilfsstoffe befeuchtet, in feuchten Zustand miteinander bspw. durch Kneten, Rühren usw. vermischt und die Mischung anschließend granuliert. Um zu einem tablettierfähigen Granulat zu gelangen, muß nach der Feuchtgranulation das enthaltene Wasser entfernt werden. Die Trockenzeit beträgt nach dem klassischen Verfahren der Hordentrocknung ca. 15 Std., während derer die Pflanzendrogen dem Luftsauerstoff als Oxidationsmittel ausgesetzt sind. Die Trocknungstemperatur des Materials liegt bei ca. 65°C bis 75°C, was den Wirkstoffgehalt nachteilig beeinflussen kann. Weiterhin kommt es aufgrund der langen Trocknungszeit und aufgrund der Verwendung von nicht-sterilem Pflanzenmaterial immer wieder zum Anwachsen von unerwünschten Keimen wie Schimmelkulturen, die bei diesen Systemvorgaben besondere Wachstumsvoraussetzungen haben.

Andererseits sind die Grenzen der Keimbelastung von pflanzlichen Drogen für Arzneimittel mit einem Gehalt an derartigen Materialen im DAB 98 (Deutsches Arzneibuch, 10. Ausgabe) festgelegt. Häufig müssen daher Drogen, obwohl sie ansich chemisch oder pharmazeutisch gesehen verwertbar wären, verworfen werden, weil sie den strengen Anforderungen der Arzneibücher nicht genügen. Um die erforderlichen Keimzahlen dennoch einhalten zu können, ist daher bspw. in der DE-A-195 47 973 vorgeschlagen worden, die pflanzlichen Drogen vor der Feuchtgranulation gemäß einem bestimmten Verfahren in einem Vakuumtrocknungssytem mit mehrschenkligem Rührwerk unter Bedingungen verminderten Druckes zu entkeimen und dadurch die Ausgangskeimzahl des in der Feuchtgranulation eingesetzen Materials zu senken. Je geringer die Ausgangskeimzahl, desto geriner auch die Keimzahl des nach der Haupttrocknung erhaltenen, tablettierfähigen Granulats. Die Trockendauer wird hierdurch jedoch nicht gesenkt, die Exposition an Luftsauerstoff ist damit unverändert.

Selbst die kürzere Trocknung mittels eines Wirbelschichtgeräts benötigt zwischen 7 und 8 Stunden bei einer auf 65°C vorgeheizten Zulufttemperatur. Gleichzeitig werden große Luftmengen benötigt (3.000 bis 5.000 cm³/Std). Somit werden in einer Schicht-Chargen-Produktion zwischen 21.000 und 40.000 cm³ vorgeheizter Luft benötigt. Andererseits ist nachgewiesen, daß Luft und insbesondere erwärmte Luft einen negativen Einfluß auf den Gehalt von Flavonoiden, etherischen Ölen, Polyphenolen und ungesättigten Fettsäuren ausübt, die gegenüber Oxidation durch den Luftsauerstoff besonders empfindlich sind. Die intensive Exposition ist daher unerwünscht. Aufgrund der ebenfalls recht langen Trocknungszeit von eben in der Regel 7 bis 8 Stunden ist daneben auch bei der Wirbelschichttrocknung die Möglichkeit der Vermehrung von aeroben Keimen gegeben, so daß auch dieses Verfahren die oben genannten Probleme aufweist.

Bei der Direkttablettierung, dem zweiten aus dem Stand der Technik bekannten Verfahren zur Herstellung von Tabletten aus getrockneten Pflanzendrogen, müssen die einzelnen Bestandteile der Tabletten, also Drogenmaterial und Träger- bzw. Hilfsstoffe, in zur Direkttablettierung geeigneter Form bspw. als Granulat vorliegen und in dieser Form miteinander aufwändig gemischt werden. Da zum Teil in den fertigen Tabletten neben den arzneilich wirksamen Bestandteilen bis zu 20 verschiedene Stoffe vorliegen können, die jeweils die Einbringung an genau definierter Stelle des Produktionsablaufes erfordern, damit die neu hinzugefügten Inhaltsstoffe im Granulat stabil bleiben, stellt die Direkttablettierung ein relativ aufwändiges Verfahren dar. Der Granulationsvorgang ist daher in der pharmazeutischen Industrie ein langwieriger, kostenintensiver Vorgang, der in unerwünschtem Maße Zeit, Energie und technisches Personal bindet.

Aufgabe der vorliegenden Erfindung ist es deshalb, ein Verfahren bereitzustellen, das schnell, auf einfache Weise, unter geringem Aufwand von Zeit, Energie und Personal, jedoch effektiv hinsichtlich der Produktverträglichkeit der einzelnen Inhalts- und Hilfsstoffe, die Herstellung eines fließfähigen, pressfertigen Arzneimittelvorprodukts zur Herstellung von Tabletten ermöglicht, welches gleichzeitig schonend für die Pflanzeninhaltsstoffe ist und adäquate Keimbelastungen erzielt.

Gelöst wird die erfindungsgemäße Aufgabe durch ein Verfahren zur Herstellung eines fließfähigen, pressfertigen Vorprodukts, bei dem getrocknetes Pflanzendrogenmaterial zusammen mit den gewünschten Zusatz-, Träger- und/oder Hilfsstoffen in einer Vakuumtrocknungsanlage mit Wasser oder einem Wasser/Lösungsmittelgemisch in einer Menge von 1 % bis 5 % befeuchtet wird, die ein sich durch eine zylindrische Misch- und Trockenkammer erstreckendes, mehrschenkliges Rührwerk mit eigenem Antrieb aufweist, wobei die Anlage erforderlichenfalls jeweils mit einem Filter, einer Rückspüleinrichtung, einem Lösungsmittelkondensator mit Nachkühler und Auffanggefäß, einem Rückkondensator und/oder einer Prozess-, Steuer- und Regeleinheit versehen ist und das feuchte Gemisch bei einer Manteltemperatur des Geräts von 20°C bis 50°C, vorzugsweise 20°C bis 40°C und am meisten bevorzugt 30°C, einer Produkttemperatur zwischen 20°C und 40°C, vorzugsweise 20°C und 30°C und am meisten bevorzugt 20°C bis 25°C und einem Druck von 20 bis 500 mbar, vorzugsweise 50 bis 200 mbar und am meisten bevorzugt 50 bis 100 mbar zum Erhalt des Vorprodukts getrocknet wird.

Handelsübliche Vakuumtrocknungsanlagen, die im erfindungsgemäßen Verfahren verwendbar sind, sind bspw. die ITUT-INOX®-Universaltrockner, z.B. die Typen ITUT 20, ITUT 50, ITUT 100 oder ITUT 2.000.

Die gewünschten Bestandteile der fertigen Tablette bzw. Arzneimittelzubereitung können gleichzeitig oder nacheinander in das Verfahren eingespeist werden. Durch gleichzeitiges Vermischen und Trocknen der Bestandteile mit Hilfe des mehrschenkligen Rührwerks muß dem Feuchtgranulat erheblich weniger Feuchtigkeit als im herkömmlichen Feuchtgranulationsverfahren zugesetzt werden. Vorzugsweise erfolgt die Befeuchtung durch Zusatz von Wasser oder einem Wasser/Lösungsmittelgemisch in einer Menge von 0,01 bis 0,05 ml/g, vorzugsweise von 0,02 bis 0,04 ml/g Feststoffe. Als Lösungsmittel können vorzugsweise niedere Alkohole wie Methanol, Ethanol, Propanol oder Glykol und Glycerin, Ketone wie Aceton oder Ether wie Diethylether, Glyme, Diglyme verwendet werden. Bevorzugt werden Wasser oder Wasser/Alkohol-Gemische eingesetzt. Außerdem wird bevorzugt hoch disperses Siliciumdioxid in den Grenzen von 1 bis 10 % zugesetzt, bezogen auf die Gesamtmasse, vorzugsweise zwischen 3 bis 6 %.

Aufgrund der geringeren Befeuchtung ist es wiederum möglich, die Trockenzeit auf Werte zwischen 30 min bis 2 Std., vorzugsweise 1 bis 1,5 Std. zu senken. Da neben der verkürzten Trocknungszeit, die zu erheblich verringerten Keimzahlen führt, die Trocknungstemperatur gesenkt und die Luftzufuhr verringert werden kann, weisen die erfindungsgemäßen fließfähigen, pressfertigen Vorprodukte einen höheren Gehalt an arzneilich wirksamen Bestandteilen im Vergleich zu nach herkömmlichen Verfahren hergestellten Feuchtgranulaten auf. Dies beruht darauf, daß die unerwünschten Auswirkungen der Trocknung an Luft eliminiert werden können. Das erfindungsgemäße Verfahren weist weiterhin den Vorteil auf, daß die Zeit zur Granulatherstellung erheblich verkürzt ist, die Herstellung erheblich vereinfacht ist und mit weniger Personal durchgeführt werden kann.

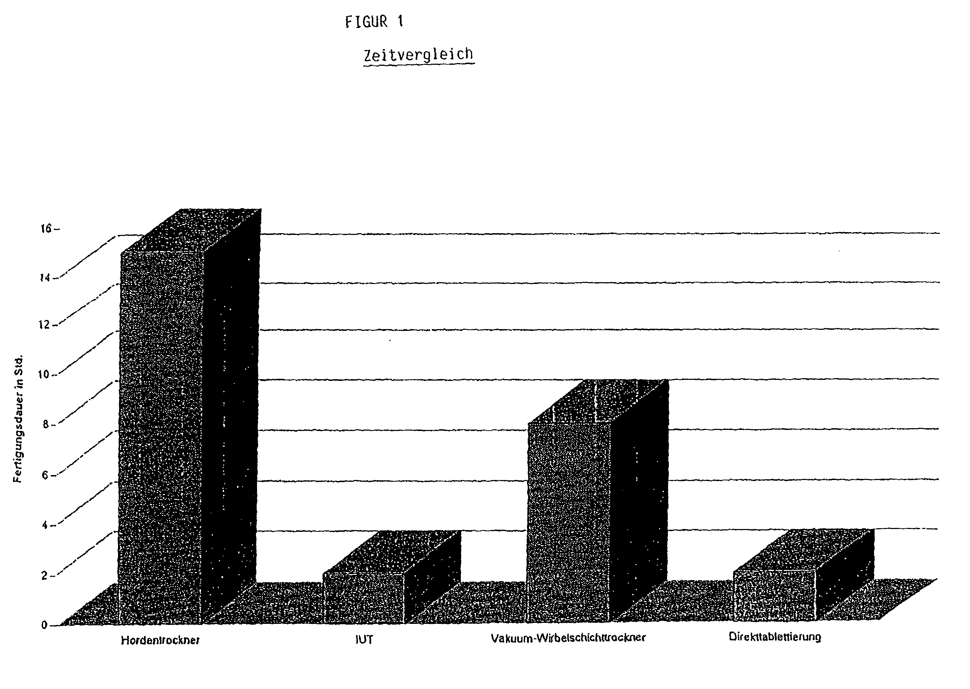

In Abb. 1 ist ein Zeitvergleich zwischen klassischer Feuchtgranulation, Wirbelschichtgranulation und erfindungsgemäßer Vakuumgranulation sowie der Direkttablettierung gezeigt. Aus der Darstellung ist ersichtlich, daß das erfindungsgemäße Verfahren hinsichtlich des Zeitvorteils mit der Direkttablettierung vergleichbar ist. Diese ist jedoch erheblich verfahrensaufwendiger und stellt wesentlich höhere Anforderungen an die Reinheit und physikalische Beschaffenheit der einzusetzenden Ausgangsmaterialien. Insgesamt bietet daher das erfindungsgemäße Verfahren Vorteile gegenüber allen Verfahren des Standes der Technik.

Bei dem erfindungsgemäß einsetzbaren Pflanzentrockenmaterial kann es sich um Material aus Blüten, Wurzeln, Rhizomen, Knollen, Blättern oder anderen Pflanzenteilen der jeweils gewünschten Pflanze handeln. Für die Drogen seien hier bspw. Arten von Althaea, Juglans, Millefolium, Centaurium, Rosmarinum, Gentiana, Primula, Rumex, Sambuco, Echinacea, Phyllanthus und Verbena wie auch Agnus Castus, Allium, insbesondere Allium cepa, Hedera helix, Hippocastanus, Curcuma, Galphimia, insbesondere Galphimia glauca, Herba Thymii oder deren Gemische angeführt. Besonders bevorzugt sind Flos Sambuci, Flos Primulae, Herba Rumicis, Radix Gentianae, Herba Verbenae oder deren Gemische, beispielsweise die zur Herstellung von Sinupret® bzw. Sinupret® forte Dragees verwendeten Gemische.

Erfindungsgemäß zu verwendende Träger-, Zuschlags-, und Hilfstoffe sind die herkömmlicherweise zur Tablettierung verwendeten Zuschlags-, Träger-, und Hilsstoffe. Insbesondere sind hier zu nennen Stärke sowie Stärkederivate, Siliciumdioxid, Lactose, Lactosemonohydrat, Cellulose sowie Cellulosederivate, Magnesiumstearat, Calciumstearat, Calciumhydrogenphosphat, PVP oder Povidon, Polyethylenglykol oder Macrogol, Mannit, Sorbit, Gelatine, Zuckeralkohole, Stearinsäure und deren Salze sowie Mischungen derselben. Siliciumdioxid allein oder im Gemisch mit einem oder mehreren der genannten Stoffe ist bevorzugt. Insbesondere sind zu nennen: Acrylderivate, Alginsäure, Alpha-octadecyl-omega-hydroxypoly-(oxyethylen)-5-sorbinsäure-H₂O, arabisches Gummi, Aromastoffe, Ascorbinsäure, Calciumcarbonat, Calciumhydrogenphosphat, Calciumphosphat, Calciumstearat, Carmellose-Natrium, Cellulose, Cellulosederivate, Dimeticon, Farbstoffe, Gelatine, Glucosesirup, hochdisperses Siliciumdioxid, Hypromellose, Kaliumbenzoat, Lactosemonohydrat, Macrogol, Magnesiumcarbonat, Magnesiumoxid (leicht), Magnesiumstearat, Maisstärke, Maisquellstärke, Mannit, Mannitol, Monound Diglyceride von Speisefettsäuren, Montanglycolwachs, Natriumbenzoat, Natriumcarbonat (wasserfrei), Natriumchlorid, Natriumhydrogencarbonat, Poly(butylmethacrylatco-(2-dimethylaminoethyl-methacrylat), Polyvidon K 25, Povidon, raffiniertes Rizinusöl, Saccharose, Saccharosemonostearat, Schellack, Sorbit, Stärke, Stärkederivate, Stearinsäure, Talkum, Titandioxid und Weinsäure.

Das erhaltende Vorprodukt kann ohne weitere Zwischenschritte zu Tabletten, Dragees oder Pellets der gewünschten Größe und Dichte verpreßt werden. Diese können anschließend gemäß herkömmlicher Verfahren mit Überzügen, Drageehüllen etc. versehen werden. Das Vorprodukt kann aber auch als solches in Kapseln bspw. aus Hart- oder Weichgelatine gefüllt, zu anderen, einzeln verpackten Formen direkt abgefüllt oder als Pulver/Granulat formuliert werden.

### Zusammensetzungsbeispiele

### 1. Sinupret® Dragees

### Zusammensetzung

| **1.** | **Wirksame Bestandteile** | **Menge** |
|---|---|---|
| | Enzianwurzel, plv. | 6.000 mg |
| | Primelblüten mit Kelch, plv. | 18.000 mg |
| | Sauerampferkraut, plv. | 18.000 mg |
| | Eisenkraut, plv. | 18.000 mg |
| | Holunderblüten, plv. | 18.000 mg |

### 2. Sonstige mögliche Bestandteile

Acrylderivate, Alginsäure, Alpha-octadecyl-omega-hydroxypoly-(oxyethylen)-5-sorbinsäure-H₂O, Arabisches Gummi, Aromastoffe, Ascorbinsäure, Calciumcarbonat, Calciumhydrogenphosphat, Calciumphosphat, Calciumstearat, Carmellose-Natrium, Cellulose, Cellulosederivate, Dimeticon, Farbstoffe, Gelatine, Glucosesirup, hochdisperses Siliciumdioxid, Hypromellose, Kaliumbenzoat, Lactosemonohydrat, Macrogol, Magnesiumcarbonat, Magnesiumoxid (leicht), Magnesiumstearat, Maisstärke, Maisquellstärke, Mannit, Mannitol, Mono- und Diglyceride von Speisefettsäuren, Montanglycolwachs, Natriumbenzoat, Natriumcarbonat (wasserfrei), Natriumchlorid, Natriumhydrogencarbonat, Poly(butylmethacrylatco-(2-dimethylaminoethyl-methacrylat), Polyvidon K 25, Povidon, raffiniertes Rizinusöl, Saccharose, Saccharosemonostearat, Schellack, Sorbit, Stärke, Stärkederivate, Stearinsäure, Talkum, Titandioxid und Weinsäure.

### 2. Sinupret forte Dragees

### Zusammensetzung

| 1. | Wirksame Bestandteile | Menge |
|---|---|---|
| | Enzianwurzel, plv. | 12.000 mg |
| | Primelblüten mit Kelch, plv. | 36.000 mg |
| | Sauerampferkraut, plv. | 36.000 mg |
| | Eisenkraut, plv. | 36.000 mg |
| | Holunderblüten, plv. | 36.000 mg |

### 2. Sonstige mögliche Bestandteile

Acrylderivate, Alginsäure, Alpha-octadecyl-omega-hydroxypoly-(oxyethylen)-5-sorbinsäure-H₂O, Arabisches Gummi, Aromastoffe, Ascorbinsäure, Calciumcarbonat, Calciumhydrogenphosphat, Calciumphosphat, Calciumstearat, Carmellose-Natrium, Cellulose, Cellulosederivate, Dimeticon, Farbstoffe, Gelatine, Glucosesirup, hochdisperses Siliciumdioxid, Hypromellose, Kaliumbenzoat, Lactosemonohydrat, Macrogol, Magnesiumcarbonat, Magnesiumoxid (leicht), Magnesiumstearat, Maisstärke, Maisquellstärke, Mannit, Mannitol, Mono- und Diglyceride von Speisefettsäuren, Montanglycolwachs, Natriumbenzoat, Natriumcarbonat (wasserfrei), Natriumchlorid, Natriumhydrogencarbonat, Poly(butylmethacrylatco-(2-dimethylaminoethyl-methacrylat), Polyvidon K 25, Povidon, raffiniertes Rizinusöl, Saccharose, Saccharosemonostearat, Schellack, Sorbit, Stärke, Stärkederivate, Stearinsäure, Talkum, Titandioxid und Weinsäure.

### Herstellungsverfahren

Die arzneilich wirksamen Bestandteile sowie die sonstigen Bestandteile der Kernmasse wurden mit einer INOX-Glatt-Mauer Anlage bei einer Manteltemperatur des Geräts von 30°C, einer Produkttemperatur zwischen 20°C und 45°C und einem Druck von 100 mbar mit einer Wassermenge von 0,3 1 (bei einem 10 kg Ansatz) befeuchtet und dann innerhalb einer Stunde getrocknet. Anschließend wurde das erfindungsgemäße Produkt mit Stearinsäure gemischt und die fließfähige, pressfertige Mischung zu Tabletten verpresst. Diese wurden gemäß herkömmlicher Verfahren mit der Drageehülle versehen.

Da die Trocknungszeit mit einer Stunde erheblich kürzer als die für das klassische Feuchtgranulierverfahren benötigten 15 Stunden war, weist das erfindungsgemäße Verfahren hinsichtlich des Durchsatzvolumens erhebliche Vorteile gegenüber der Feuchtgranulation auf.

## Patentansprüche

1. Verfahren zur Herstellung eines fließfähigen, pressfertigen Arzneimittelvorprodukts, bei dem getrocknetes Pflanzendrogenmaterial zusammen mit den gewünschten Zusatz-, Träger- und/oder Hilfsstoffen in einer Vakuumtrocknungsanlage mit Wasser oder einem Wasser/Lösungsmittel-Gemisch in einer Menge von 1 % bis 5 % befeuchtet wird, wobei die Vakuumtrocknungsanlage ein sich durch eine zylindrische Misch- und Trockenkammer erstreckendes, mehrschenkliges Rührwerk mit eigenen Antrieb aufweist, und das feuchte Gemisch bei einer Manteltemperatur des Geräts von 20°C bis 50°C, einer Produkttemperatur von 20°C bis 45°C und einem Druck von 20 bis 500 mbar zum Erhalt des Vorprodukts getrocknet wird.

2. Verfahren gemäß Anspruch 1, bei dem die Vakuumtrocknungsanlage weiterhin einen Filter, eine Rückspüleinrichtung, einen Lösungsmittelkondensator mit Nachkühler und Auffanggefäß, einem Rückkondensator und/oder eine Prozess-, Steuer- und Regeleinheit aufweist.

3. Verfahren gemäß Anspruch 1 oder 2, **dadurch gekennzeichnet, dass** die Manteltemperatur des Geräts 20°C bis 40°C, die Produkttemperatur 20°C bis 30°C und der Druck 50 bis 200 mbar beträgt.

4. Verfahren gemäß einem der vorstehenden Ansprüche, **dadurch gekennzeichnet, dass** die Manteltemperatur des Geräts 30°C, die Produkttemperatur 20°C bis 25°C und der Druck 50 bis 100 mbar beträgt.

5. Verfahren nach einem der vorstehenden Ansprüche, bei dem dem Pflanzendrogenmaterial hochdisperses Siliciumdioxid in einer Menge von 1 % bis 10 %, bezogen auf die Gesamtmenge zugesetzt wird.

6. Verfahren gemäß einem der vorstehenden Ansprüche, **dadurch gekennzeichnet, dass** es sich bei dem eingesetzten Pflanzentrockenmaterial um Material aus Blüten, Wurzeln, Rhizomen, Knollen, Blättern oder anderen Pflanzenteilen der jeweils gewünschten Pflanze oder Pflanzengemisch handelt.

7. Verfahren gemäß Anspruch 6, **dadurch gekennzeichnet, dass** es sich bei dem Pflanzentrockenmaterial um Drogen von Althaeae, Juglandis, Millefolii, Centaurii, Rosmarinum, Gentiana, Primula, Rumex, Sambuco, Echinacea, Phyllanthus und Verbena wie auch Agnus Castus, Allium, Hedera helix, Hippocastanus, Curcuma, Galphimia und Herba Thymii sowie weiteren Pflanzentrockenmaterialien oder deren Gemische handelt.

8. Verfahren gemäß Anspruch 7, **dadurch gekennzeichnet, dass** es sich bei dem Pflanzendrogen um Flos Sambuci, Flos Primulae, Herba Rumicis, Radix Althaeae, Folium Rosmarini, Folium Juglandis, Herba Millefolii, Herba Centaurii, Herba Thymii, Radix Gentianae, Herba Verbenae sowie weiteren Pflanzentrockenmaterialien oder deren Gemische handelt.

9. Fließfähiges, pressfertiges Arzneimittelvorprodukt, erhalten gemäß einem der vorstehenden Ansprüche.

10. Fließfähiges, pressfertiges Arzneimittelvorprodukt gemäß Anspruch 9, **dadurch gekennzeichnet, dass** es sich bei dem verwendeten Pflanzentrockenmaterial um eine Mischung von gepulvertem Ampferkraut, gepulvertem Eisenkraut, gepulverter Enzianwurzel, gepulverten Holunderblüten und gepulverten Schlüsselblumen handelt.

## Claims

1. Method for manufacturing a flowable, press-ready semi-finished pharmaceutical product in which a dried plant/vegetable medicinal material together with the required additive, carrier and/or adjuvant materials is moistened with a quantity of 1 % to 5 % of water or a water/solvent mixture in a vacuum drying equipment, whereby the vacuum drying equipment has a multi-spindle stirrer with its own drive extending through a cylindrical mixing and drying compartment, and the moist mixture is dried at an equipment jacket temperature of 20°C to 50°C, a product temperature of 20°C to 45°C and a pressure of 20 to 500 mbar to obtain the semi-finished product.

2. Method according to Claim 1, in which the vacuum drying equipment also has a filter, a back-flush device, a solvent condenser with aftercooler and collection vessel, a reflux condenser and/or a process, control and regulator unit.

3. Method according to Claim 1 or 2, **characterized in that** the jacket temperature of the equipment is 20°C to 40°C, the product temperature is 20°C to 30°C and the pressure is 50 to 200 mbar.

4. Method according to one of the foregoing Claims, **characterized in that** the jacket temperature of the equipment is 30°C, the product temperature is 20°C to 25°C and the pressure is 50 to 100 mbar.

5. Method according to one of the foregoing Claims, in which highly dispersed silicon dioxide in a quantity from 1 % to 10 % relative to the total amount is added to the plant/vegetable medicinal material.

6. Method according to one of the foregoing Claims, **characterized in that** the dried plant material used involves material from the flowers/blossoms, roots, rhizomes, tubers, leaves or other plant parts of the respective required plant or plant mixture.

7. Method according to Claim 6, **characterized in that** the dried plant material involves medications from Althaeae, Juglandaceae, Millefolii, Centaureae, Rosmarinum, Gentiana, Primula, Rumex, Sambucus, Echinacea, Phyllanthus and Verbena and also Agnus Castus, Allium, Hedera helix, Hippocastanus, Curcuma, Galphimia and Herba Thymii as well as other dried plant/vegetable materials or mixtures thereof.

8. Method according to Claim 7, **characterized in that** the plant medicines involve Flos Sambuci, Flos Primulae, Herba Rumicis, Radix Althaeae, Folium Rosmarini, Folium Juglandis, Herba Millefolii, Herba Centaurii, Herba Thymii, Radix Gentianae, Herba Verbenae as well as other dried plant/vegetable materials or mixtures thereof.

9. Flowable, press-ready semi-finished pharmaceutical product obtained according to one of the foregoing Claims.

10. Flowable, press-ready semi-finished pharmaceutical product according to Claim 9, **characterized in that** the dried plant/vegetable material used involves a mixture of powdered dock/sorrel, powdered vervain, powdered gentian root, powdered elderflowers and powdered cowslip/primula flowers.

## Revendications

1. Procédé pour la préparation d'un précurseur de médicament apte à l'écoulement, prêt à être comprimé, dans lequel un matériau de drogues végétales séché est humidifié avec les additifs, supports et/ou adjuvants souhaités dans une installation de séchage sous vide avec de l'eau ou un mélange eau/solvant en une quantité de 1 % à 5 %, dans lequel l'installation de séchage sous vide présente un agitateur à plusieurs pales avec commande autonome, s'étendant à travers une chambre de mélange et de séchage cylindrique, et le mélange humide est séché à une température de manteau de l'appareil de 20°C à 50°C, une température de produit de 20°C à 45°C et une pression de 20 à 500 mbar pour l'obtention du précurseur du produit.

2. Procédé selon la revendication 1, dans lequel le dispositif de séchage sous vide présente en outre un filtre, un dispositif de lavage à contre-courant, un condensateur de solvants avec réfrigérant et récipient de récupération, un condensateur à reflux et/ou une unité de procédé, de commande et de régulation.

3. Procédé selon la revendication 1 ou 2, **caractérisé en ce que** la température de manteau de l'appareil est entre 20°C et 40°C, la température du produit entre 20°C et 30°C et la pression entre 50 et 200 mbar.

4. Procédé selon une des revendications précédentes, **caractérisé en ce que** la température de manteau de l'appareil s'élève à 30°C, la température du produit entre 20°C et 25°C et la pression entre 50 et 100 mbar.

5. Procédé selon une des revendications précédentes, dans lequel on ajoute au matériau de drogues végétales du dioxyde de silicium hautement dispersé en une quantité de 1 % à 10 %, par rapport à la quantité totale.

6. Procédé selon une des revendications précédentes, **caractérisé en ce qu'**il s'agit pour le matériau de drogues végétales mis en oeuvre de matériau provenant de fleurs, de racines, de rhizomes, de tubercules, de feuilles ou d'autres parties végétales respectivement de plante ou de mélange de plantes souhaités.

7. Procédé selon la revendication 6, **caractérisé en ce qu'**il s'agit pour le matériau sec végétal de drogues de Althaeae, Juglandis, Millefolii, Centaurii, Rosmarinum, Gentiana, Primula, Rumex, Sambucco, Echinacea, Phyllanthus et Verbena et aussi Agnus Castus, Allium, Hedera helix, Hippocastanus, Curcuma, Galphimia et Herba Thymii ainsi que d'autres matériaux secs végétaux ou leurs mélanges.

8. Procédé selon la revendication 7, **caractérisé en ce qu'**il s'agit pour les drogues végétales de Flos Sambuci, Flos Primulae, Herba Rumicis, Radix Althaeae, Folium Rosmarini, Folium Juglandis, Herba Millefolii, Herba Centaurii, Herba Thymii, Radix Gentianae, Herba Verbenae ainsi que d'autres matériaux secs végétaux ou leurs mélanges.

9. Précurseur de médicament apte à l'écoulement, prêt à être comprimé, obtenu selon l'une des revendications précédentes.

10. Précurseur de médicament apte à l'écoulement, prêt à être comprimé selon la revendication 9, **caractérisé en ce qu'**il s'agit pour le matériau sec végétal utilisé d'un mélange d'oseille en poudre, de verveine en poudre, de racine de gentiane en poudre, de fleurs de sureau en poudre et de fleurs de primevères en poudre.
